# EUROPÄISCHE PATENTANMELDUNG

(11) **EP 1 440 973 A2**
(43) Veröffentlichungstag der Anmeldung: **28.07.2004**
(21) Anmeldenummer: 04005011.4
(22) Anmeldetag: 18.11.1996
(51) Int. Cl.: C07D 417/06, C07D 493/04, C07D 497/18, A61K 31/425, A01N 43/78, A61P 35/00

(54) **Epothilonderivate, Herstellung und Mittel**

(30) Priorität: 17.11.1995 DE 19542986; 25.09.1996 DE 19639456
(62) Teilanmeldung aus: 01127352.1
(71) Anmelder: Gesellschaft für Biotechnologische Forschung mbH (GBF), 38124 Braunschweig (DE)
(72) Erfinder: Höfle, Gerhard, Prof. Dr., 38124 Braunschweig (DE); Kiffe, Michael, Dr., 38124 Braunschweig (DE)
(74) Vertreter: Boeters, Hans Dietrich, Dr.

(57) **Zusammenfassung**

Die Erfindung betrifft Epothilon-Derivate als Lactone sowie offenkettige Epothilon-Derivate, Herstellungsverfahren und Mittel mit einem Gehalt an den Derivaten.

## Beschreibung

Die vorliegende Erfindung betrifft allgemein Epothilonderivate und deren Verwendung zur Herstellung von Arzneimitteln. Insbesondere betrifft die vorliegende Erfindung die Herstellung der Epothilonderivate der nachfolgend dargestellten allgemeinen Formeln 1 bis 7 sowie deren Verwendung zur Herstellung von therapeutischen Mitteln und Mitteln für den Pflanzenschutz.

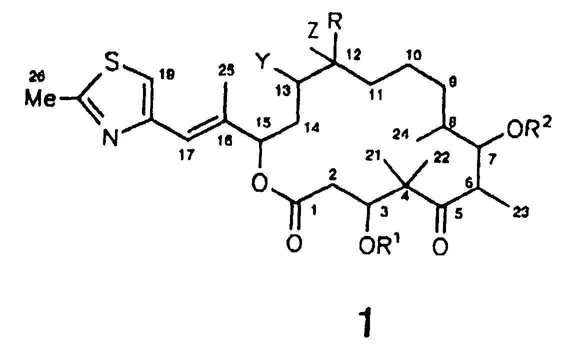

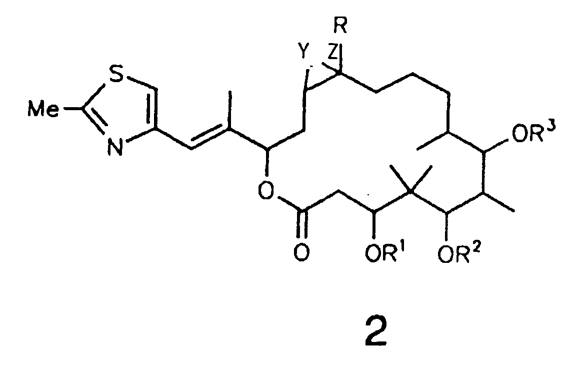

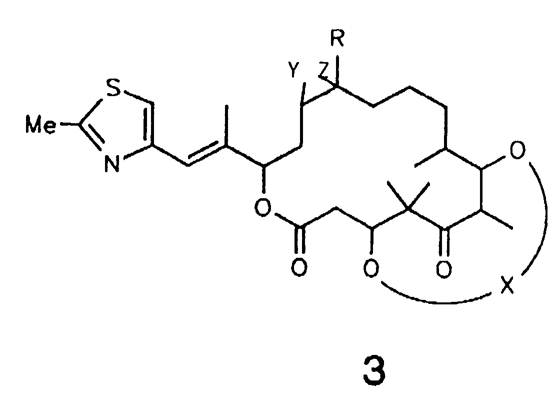

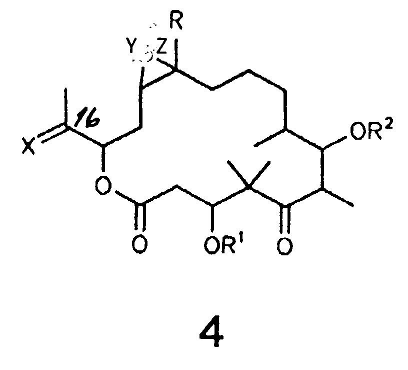

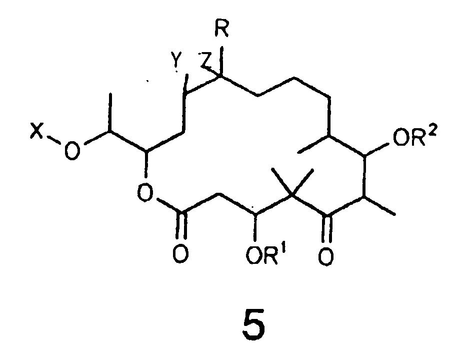

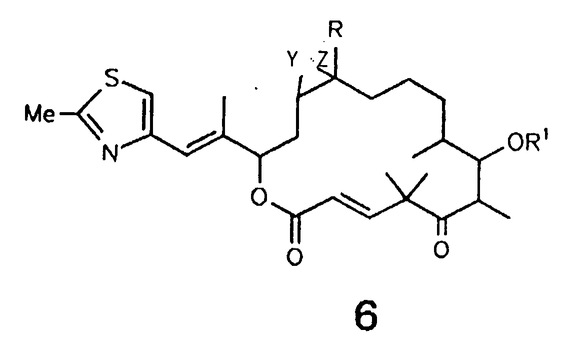

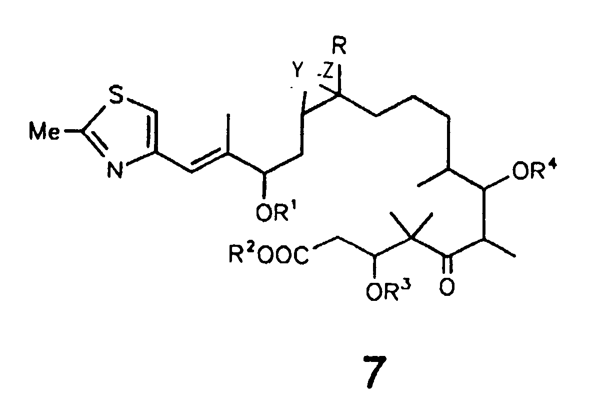

In den vorstehenden **Formeln 1 bis Formel 7** bedeuten:
R = H, C₁₋₄-Alkyl;
R¹, R², R³, R⁴, R⁵ = H, C₁₋₆-Alkyl,
C₁₋₆-Acyl-Benzoyl,
C₁₋₄-Trialkylsilyl,
Benzyl,
Phenyl,
C₁₋₆-Alkoxy-,
C₆-Alkyl-, Hydroxy- und Halogen-
substituiertes Benzyl bzw. Phenyl;
wobei auch zwei der Reste R¹ bis R⁵ zu der Gruppierung -(CH₂)ₙ- mit n = 1 bis 6 zusammentreten können und es sich bei den in den Resten enthaltenen Alkyl- bzw. Acylgruppen um gradkettige oder verzweigte Reste handelt;
Y und Z sind entweder gleich oder verschieden und stehen jeweils für Wasserstoff, Halogen, wie F, Cl, Br oder J, Pseudohalogen, wie -NCO, -NCS oder -N₃, OH, O-(C₁₋₆)-Acyl, O-(C₁₋₆)-Alkyl, O-Benzoyl. Y und Z können auch das O-Atom eines Epoxides sein,
wobei Epothilon A und B nicht beansprucht werden, oder eine der C-C-Bindungen einer C=C-Doppelbindung bilden.

In der **Formel 3** steht X allgemein für -C(O)-, -C(S)-, -S(O)-, -CR¹R²-, wobei R¹ und R² die Bedeutung haben wie oben angegeben, und -SiR₂-, wobei R die Bedeutung hat wie oben angegeben.

In der **Formel 4** bedeutet X Sauerstoff, NOR³, N-NR⁴R⁵, und N-NHCONR⁴R⁵, wobei die Reste R³ bis R⁵ die oben angegebene Bedeutung haben.

In der **Formel 5** bedeutet X Wasserstoff, C₁₋₁₈-Alkyl, C₁₋₁₈-Acyl, Benzyl, Benzoyl und Cinnamoyl.

Für Epothilon A und B sei verwiesen auf DE-A-41 38 042. Verbindungen gemäß der allgemeinen **Formel 1** sind ausgehend von Epothilon A und B sowie von deren 3-O- und/oder 7-O-geschützten Derivaten durch Öffnung des 12,13-Epoxids zugänglich. Werden dazu Hydrogenwasserstoffsäuren in einem bevorzugt nicht wässrigen Lösungsmittel eingesetzt, wobei man die Halogenhydrine X = Hal, Y = OH und Y = OH, Y = Hal erhält. Protonensäuren wie z.B. Toluolsulfonsäure und Trifluoressigsäure führen in Gegenwart von Wasser zu 12,13-Diolen, die anschließend nach Standardverfahren acyliert (z.B. mit Carbonsäureanhydriden und Pyridin oder Triethylamin/DMAP) oder alkyliert (Alkylhalogenide und Silberoxid) werden. Die 3- und 7-Hydroxygruppen können dazu vorübergehend als Formiat (Abspaltung mit NH₃/MeOH) oder p-Methoxybenzylether (Abspaltung mit DDQ) geschützt werden.

Verbindungen gemäß der allgemeinen **Formel 2** sind aus Epothilon A und B sowie deren 3-O- und/oder 7-O-geschützten Derivaten durch Reduktion, z.B. mit NaBH₄ in Methanol erhältlich. Sind dabei 3-OH und/oder 7-OH reversibel geschützt, so können nach Acylierung oder Alkylierung und Entfernen der Schutzgruppen 5-O-monosubstituierte, 3,5- oder 5,7-O-disubstituierte Derivate der allgemeinen **Formel 2** erhalten werden.

Umsetzungen von Epothilon A und B mit bifunktionellen elektrophilen Reagenzien, wie (Thio)Phosgen, (Thio)Carbonyldimidazol, Thionylchlorid oder Dialkylsilyldichloriden bzw. -bistriflaten ergeben Verbindungen der allgemeinen **Formel 3**. Als Hilfsbasen dienen dabei Pyridin, Trialkylamine, ggf. zusammen mit DMAP bzw. 2,6-Lutidin in einem nichtprotischen Lösungsmittel. Die 3,7-Acetale der allgemeinen **Formel 3** entstehen durch Umacetalisierung z.B. von Dimethylacetalen in Gegenwart eines sauren Katalysators.

Verbindungen gemäß der allgemeinen Formel 4 werden aus Epothilon A und B oder ihren 3-O- und/oder 7-O-geschützten Derivaten durch Ozonolyse und reduktive Aufarbeitung, z.B. mit Dimethylsulfid, erhalten. Die C-16-Ketone können anschließend nach dem Fachmann geläufigen Standardverfahren in Oxime, Hydrazone oder Semicarbazone umgewandelt werden. Sie werden weiterhin durch Wittig-, Wittig-Horner-, Julia- oder Petersen-Olefinierung in C-16/C-17-Olefine überführt.

Durch Reduktion der C-16-Ketogruppe, z.B. mit einem Aluminiumoder Borhydrid, sind die 16-Hydroxyderivate gemäß der allgemeinen **Formel 5** erhältlich. Diese können, wenn 3-OH und 7-OH mit entsprechenden Schutzgruppen versehen sind, selektiv acyliert oder alkyliert werden. Die Freisetzung der 3-OH- und 7-OH-Gruppen erfolgt z.B. bei O-Formyl durch NH₃/MeOH, bei O-p-Methoxybenzyl durch DDQ.

Die Verbindungen der allgemeinen **Formel 6** werden aus Derivaten von Epothilon A und B erhalten, bei denen die 7-OH-Gruppe durch Acyl- oder Ethergruppen geschützt ist, in dem die 3-OH-Gruppe z.B. formyliert, mesyliert oder tosyliert und anschließend durch Behandlung mit einer Base z.B. DBU eliminiert wird. Die 7-OH-Gruppe kann wie oben beschrieben freigesetzt werden.

Verbindungen der allgemeinen **Formel 7** werden aus Epothilon A und B oder deren 3-OH- und 7-OH-geschützten Derivaten durch basische Hydrolyse erhalten, z.B. mit NaOH in MeOH oder MeOH/Wasser. Vorzugsweise werden Verbindungen der allgemeinen **Formel 7** aus Epothilon A oder B oder deren 3-OH- oder 7-OH-geschützten Derivaten durch enzymatische Hydrolyse erhalten, insbesondere mit Esterasen oder Lipasen. Die Carboxylgruppe kann mit Diazoalkanen nach Schutz der 19-OH-Gruppe durch Alkylierung in Ester umgewandelt werden.

Ferner können Verbindungen der Formel 7 durch Lactonisierung nach den Methoden von Yamaguchi (Trichlorbenzoylchlorid/DMAP), Corey (Aldrithiol/Triphenylphosphin) oder Kellogg (omega-Bromsäure/Caesiumcarbonat) in Verbindung der **Formel 2** umgewandelt werden. Einschlägige Arbeitsmethoden finden sich bei

Inanaga et al. in Bull. Chem. Soc. Japan, 52 (**1979**) 1989; Corey & Nicolaou in J. Am. Chem. Soc., 96 (**1974**) 5614; und Kruizinga & Kellogg in J. Am. Chem. Soc., 103 (**1981**) 5183.

Zur Herstellung der erfindungsgemäßen Verbindungen kann man auch von Epothilon C oder D ausgehen, wobei zur Derivatisierung auf die vorstehend beschriebenen Derivatisierungsmethoden verwiesen werden kann. Dabei kann man die 12,13-Doppelbindung selektiv hydrieren, beispielsweise katalytisch oder mit Diimin; oder epoxidieren, beispielsweise mit Dimethyldioxiran oder einer Persäure; oder in die Dihalogenide, Dipseudohalogenide oder Diazide umwandeln.

Die Erfindung betrifft ferner Mittel für den Pflanzenschutz in Landwirtschaft, Forstwirtschaft und/oder Gartenbau, bestehend aus einer oder mehreren der vorstehend aufgeführten Epothilonderivate bzw. bestehend aus einem oder mehreren der vorstehend aufgeführten Epothilonderivate neben einem oder mehreren üblichen Träger(n) und/oder Verdünnungsmittel(n).

Schließlich betrifft die Erfindung therapeutische Mittel, bestehend aus einer oder mehreren der vorstehend aufgeführten Verbindungen oder einer oder mehreren der vorstehend aufgeführten Verbindungen neben einem oder mehreren üblichen Träger(n) und/oder Verdünnungsmittel(n). Diese Mittel können insbesondere cytotoxische Aktivitäten zeigen und/oder Immunsuppression bewirken und/oder zur Bekämpfung maligner Tumore eingesetzt werden, wobei sie besonders bevorzugt als Cytostatika verwendbar sind.

Die Erfindung wird im folgenden durch die Beschreibung von einigen ausgewählten Ausführungsbeispielen näher erläutert und beschrieben.

### Beispiele

### Beispiel 1:

### Verbindung 1a

20 mg (0.041 mmol) Epothilon A werden in 1 ml Aceton gelöst, mit 50 µl (0.649 mmol) Trifluoressigsäure versetzt und über Nacht bei 50 °C gerührt. Zur Aufarbeitung wird das Reaktionsgemisch mit 1 M Phosphatpuffer pH 7 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes erfolgt mit Hilfe der präparativen Schichtchromatographie (Laufmittel: Dichlormethan/Aceton, 85 : 15).
Ausbeute: 4 mg (19 %) Isomer I
4 mg (19 %) Isomer II

### Isomer I

R_{f} (Dichlormethan/Aceton, 85 : 15): 0.46
IR (Film): ny = 3440 (m, b, Sch), 2946 (s, Sch), 1734 (vs), 1686 (m), 1456 (m), 1375 (w), 1256 (s, Sch), 1190 (w, b, Sch), 1071 (m, Sch), 884 (w), 735 (w) cm⁻¹.
MS (20/70 eV): m/e (%) = 493 (43 [M-H₂O]⁺), 394 (47), 306 (32), 206 (30), 181 (40), 166 (72), 139 (100), 113 (19), 71 (19), 57 (24), 43 (24).
Hochauflösung: C₂₆H₃₉O₆NS ber.: 493.2498 für [M-H₂O]⁺ gef.: 493.2478

### Isomer II

R_{f} (Dichlormethan/Aceton, 85 : 15): 0.22
IR (Film): ny = 3484 (s, b, Sch), 2942 (vs, Sch), 1727 (vs), 1570 (w), 1456 (m), 1380 (m), 1265 (s), 1190 (w), 1069 (m), 975 (w), cm⁻¹.
MS (20/70 eV): m/e (%) = 493 (21 [M-H₂O]⁺), 394 (12), 306 (46), 206 (37), 181 (63), 166 (99), 139 (100), 113 (21), 71 (23), 57 (33), 43 (28).
Hochauflösung : C₂₆H₃₉O₆NS ber.: 493.2498 für [M-H₂O]⁺
gef.: 493.2475

### Beispiel 2:

### Verbindung 1b

55 mg (0.111 mmol) Epothilon A werden in 0.5 ml Tetrahydrofuran gelöst, mit 0.S ml 1 N Salzsäure versetzt und 30 Minuten bei Raumtemperatur gerührt. Anschließend wird mit 1 N Phosphatpuffer pH 7 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes erfolgt mit Hilfe der präparativen Schichtchromatographie (Laufmittel: Dichlormethan/Methanol, 90 : 10). Ausbeute: 19 mg (32 %)
R_{f} (Dichlormethan/Methanol, 90 : 10): 0.46
IR (Film): ny = 3441 (s, br, Sch), 2948 (s, Sch), 1725 (vs, Sch), 1462 (m), 1381 (w), 1265 (m), 1154 (w), 972 (m, br, Sch) cm⁻¹.
UV (Methanol): lambdaₘₐₓ (lg epsilon) = 210 (4.29), 248 (4.11) nm.
MS (20/70 eV): m/e (%) = 529 (13 [M⁺]), 494 (10), 342 (38), 306 (23), 194 (32), 164 (100), 140 (31), 113 (15), 57 (16).
Hochauflösung: C₂₆H₄₀O₆ClNS ber.: 529.2265 für [M⁺],
gef.: 529.2280

### Beispiel 3:

### Verbindung 1c

25 mg (0.047 mmol) 12-Chlor-13-hydroxy-epothilon A (1b) werden in 1 ml Dichlormethan gelöst, mit 29 mg (0.235 mmol) Dimethylaminopyridin, 151 µl (1.081 mmol) Triethylamin und 20 µl (0.517 mmol) 98 %-iger Ameisensäure versetzt. Das Reaktionsgemisch wird mit Eis/Natriumchlorid abgekühlt. Nach Erreichen von -15 °C werden dem Reaktionsgemisch 40 µl (0.423 mmol) Essigsäureanhydrid zugegeben und 70 Minuten bei -15 °C gerührt. Nachdem ein Dünnschichtchromatogramm keinen vollständigen Umsatz anzeigt, werden dem Reaktionsgemisch weitere 6 mg (0.047 mmol) Dimethylaminopyridin, 7 µl (0.047 mmol) Triethylamin, 2 µl 98 %-ige Ameisensäure (0.047 mmol) und 4 µl (0.047 mmol) Essigsäureanhydrid zugesetzt und 60 Minuten gerührt. Zur Aufarbeitung wird das Reaktionsgemisch auf Raumtemperatur erwärmt, mit 1 M Phosphatpuffer pH 7 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes erfolgt mit Hilfe der präparativen Schichtchromatographie (Laufmittel: Dichlormethan/Aceton, 90 : 10). Ausbeute: 5 mg (18 %)
R_{f} (Dichlormethan/Aceton. 90 : 10): 0.67
IR (Film): ny = 3497 (w, b, Sch), 2940 (s, b, Sch), 1725 (vs), 1468 (m, b, Sch), 1379 (m), 1265 (s), 1253 (s), 1175 (vs), 972 (m, b, Sch), 737 (s) cm⁻¹
MS (20/70 eV): m/e (%) = 613 (9 [M⁺]), 567 (43), 472 (63), 382 (23), 352 (21), 164 (100), 151 (33), 96 (31), 69 (17), 44 (26).
Hochauflösung: C₂₉H₄₀O₉NSCl ber.: 613.2112 für [M⁺]
gef.: 613.2131

### Beispiel 4:

### Verbindung 1d

10 mg (0.020 mmol) Epothilon B werden in 0.5 ml Tetrahydrofuran gelöst, mit 0.5 ml 1 N Salzsäure versetzt und 30 Minuten bei Raumtemperatur gerührt. Anschließend wird mit 1 M Phosphatpuffer pH 7 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes erfolgt mit Hilfe der präparativen Schichtchromatographie (Laufmittel: Dichlormethan/Aceton, 85 : 15).
Ausbeute: 1 mg (9 %)
R_{f} (Dichlormethan/Aceton, 85 : 15): 0.38
MS (20/70 eV): m/e (%) = 543 (3 [M⁺]), 507 (14), 320 (19), 234 (9), 194 (17), 182 (23), 164 (100), 140 (22), 113 (14), 71 (13).
Hochauflösung: C₂₇H₄₂O₆NSCl ber.: 543.2421 für [M⁺]
gef.: 543.2405

### Beispiel 5:

### Verbindung 2a

100 mg (0.203 mmol) Epothilon A werden in 4 ml Tetrahydrofuran/1 M Phosphatpuffer pH 7 (1 : 1) gelöst und solange mit Natriumborhydrid (150 mg = 3.965 mmol) versetzt bis das Edukt laut Dünnschichtchromatogramm vollständig abreagiert ist. Anschließend wird mit 1 M Phosphatpuffer pH 7 verdünnt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes erfolgt durch Kieselchromatographie (Laufmittel: Dichlormethan/Aceton, 95 : 5 - grad - nach Dichlormethan/Aceton, 85 : 15).
Ausbeute: (20 %)
R_{f} (Dichlormethan/Aceton, 75 : 25): 0.27
IR (Film): ny = 3413 (s, b, Sch), 2965 (vs, Sch), 1734 (vs), 1458 (m, b, Sch), 1383 (m, Sch), 1264 (s, b, Sch), 1184 (m, b, Sch), 1059 (s, Sch), 966 (s), 885 (w), 737 (m) cm⁻¹
MS (20/70 eV): m/e (%) = 495 (6 [M⁺]), 477 (8), 452 (12), 394 (9), 364 (16), 306 (49), 194 (19), 178 (35), 164 (100), 140 (40), 83 (21), 55 (27).
Hochauflösung: C₂₆H₄₁O₆NS ber.: 495.2655 für [M⁺]
gef.: 495.2623

### Beispiel 6:

### Verbindung 3a-d (a-d sind Stereoisomere)

100 mg (0.203 mmol) Epothilon werden in 3 ml Pyridin gelöst, mit 50 µl (0.686 mmol) Thionylchlorid versetzt und 15 Minuten bei Raumtemperatur gerührt. Anschließend wird mit 1 M Phosphatpuffer pH 7 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes und Trennung der vier Stereoisomeren **3a-d** erfolgt mit Hilfe der präparativen Schichtchromatographie (Laufmittel: Toluol/Methanol, 90 : 10).

### Verbindung 3a

Ausbeute: 4 mg (12 %)
R_{f} (Toluol/Methanol, 90 : 10) : 0.50
IR (Film): ny = 2961 (m, b, Sch), 1742 (vs), 1701 (vs), 1465 (m, Sch), 1389 (m, Sch), 1238 (s, Sch), 1210 (vs, Sch), 1011 (s, Sch), 957 (s, b, Sch), 808 (m, Sch), 768 (s, Sch) cm⁻¹
UV (Methanol): lambdaₘₐₓ (lg epsilon) = 210 (4.50), 248 (4.35) nm.
MS (20/70 eV): m/e (%) = 539 (40 [M⁺]), 457 (22), 362 (16), 316 (27), 222 (30), 178 (30), 164 (100), 151 (43), 96 (38), 69 (29), 55 (28), 43 (20).
Hochauflösung: C₂₆H₃₇O₇NS₂ ber.: 539.2011 für [M⁺]

### Verbindung 3b

Ausbeute: 14 mg (13 %)
R_{f} (Toluol/Methanol, 90 : 10): 0.44
IR (Film): ny = 2963 (s, br, Sch), 1740 (vs), 1703 (s), 1510 (w), 1464 (m, br, Sch), 1389 (m, Sch), 1240 (s, br, Sch), 1142 (m), 1076 (w), 1037 (w), 1003 (m), 945 (s, br, Sch), 806 (m, Sch), 775 (s), 737 (m) cm⁻ 1
UV (Methanol): lambdaₘₐₓ (lg epsilon) = 211 (4.16), 250 (4.08) nm.
MS (20/70 eV): m/e (%) = 539 (27 [M⁺]), 475 (17), 322 (41), 306 (67), 222 (16), 206 (17), 194 (19), 178 (32), 164 (100), 151 (33), 125 (18), 113 (15), 96 (39), 81 (23), 64 (58), 57 (42), 41 (19).
Hochauflösung: C₂₆H₃₇O₇NS₂ ber.: 539.2011 für [M⁺]
gef.: 539.1998

### Verbindung 3c

Ausbeute: 4 mg (4 %)
R_{f} (Toluol/Methanol, 90 : 10): 0.38
MS (20/70 eV): m/e (%) = 539 (51 [M⁺]), 322 (22), 306 (53), 222 (36), 178 (31), 164 (100), 151 (41), 96 (25), 81 (20), 69 (26), 55 (25), 41 (25).
Hochauflösung: C₂₆H₃₇O₇NS₂ ber.: 539.2011 für [M⁺]
gef.: 539.2001

### Verbindung 3d

Ausbeute: 1 mg (1 %)
R_{f} (Toluol/Methanol, 90 : 10): 0.33
MS (20/70 eV): m/e (%) = 539 (69 [M⁺]), 322 (35), 306 (51), 222 (41), 178 (31), 164 (100), 151 (46), 96 (31), 81 (26), 69 (34), 55 (33), 41 (35)
Hochauflösung: C₂₆H₃₇O₇NS₂ ber.: 539.2011 für [M⁺]
gef.: 539.1997

### Beispiel 7:

### Verbindung 4a

10 mg (0.020 mmol) Epothilon A werden in 2 ml Dichlormethan gelöst, auf -70 °C abgekühlt und anschließend 5 Minuten mit Ozon bis zur schwachen Blaufärbung behandelt. Das resultierende Reaktionsgemisch wird anschließend mit 0.5 ml Dimethylsulfid versetzt und auf Raumtemperatur erwärmt. Zur Aufarbeitung wird das Reaktionsgemisch vom Lösungsmittel befreit und schließlich durch präparative Schichtchromatographie (Laufmittel Dichlormethan/Aceton/Methanol, 85 : 10 : 5) gereinigt.
Ausbeute: 5 mg (64 %)
R_{f} (Dichlormethan/Aceton/Methanol, 85 : 10 : 5): 0.61
IR (Film): ny = 3468 (s, br, Sch), 2947 (s, br, Sch), 1734 (vs, Sch), 1458 (w), 1380 (w), 1267 (w), 1157 (w) , 1080 (w), 982 (w) cm⁻¹.
UV (Methanol): lambdaₘₐₓ (lg epsilon) = 202 (3.53) nm.
MS (20/70 eV): m/e (%) = 398 (2 [M⁺]), 380 (4), 267 (14), 249 (17), 211 (20), 193 (26), 171 (34), 139 (34), 111 (40), 96 (100), 71 (48), 43 (50).
Hochauflösung: C₂₁H₃₄O₇ ber.: 398.2305 für [M⁺]
gef.: 398.2295

### Beispiel 8:

### Verbindung 6a

10 mg (0.018 mmol) 3,7-Di-O-formyl-epothilon A werden in 1 ml Dichlormethan gelost, mit 27 µl (0.180 mmol)

1,8-Diazabicyclo[5.4.0]undec-7-en (DBU) versetzt und 60 Minuten bei Raumtemperatur gerührt.

Zur Aufarbeitung wird das Reaktionsgemisch mit 1 M Natriumdihydrogenphosphat-Puffer pH 4.5 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Nach Beseitigung des Lösungsmittel wird das resultierende Rohprodukt in 1 ml Methanol gelöst, mit 200 µl einer ammoniakalischen Methanollösung (2 mmol NH₃/ml Methanol) versetzt und über Nacht bei Raumtemperatur gerührt. Zur Aufarbeitung wird das Lösungsmittel im Vakuum entfernt.
Ausbeute: 4 mg (22 %)
R_{f} (Dichlormethan/Aceton, 85 : 15): 0.46
IR (Film): ny = 3445 (w, br, Sch), 2950 (vs, br, Sch), 1717 (vs, Sch), 1644 (w), 1466 (m, Sch), 1370 (m, SCh), 1267 (s, br, Sch), 1179 (s, Sch), 984 (s, Sch), 860 (w), 733 (m) cm⁻¹
UV (Methanol): lambdaₘₐₓ (lg epsilon) = 210 (4.16) nm.
MS (20/70 eV): m/e (%) = 475 (28 [M⁺]), 380 (21), 322 (37), 318 (40), 304 (66), 178 (31), 166 (100), 151 (29), 140 (19), 96 (38), 81 (20), 57 (26).
Hochauflösung: C₂₆H₃₇O₅NS ber.: 475.2392 für [M⁺]
gef. 475.2384

### Beispiel 9:

### Verbindung 6b

50 mg (0.091 mmol) 3,7-Di-O-formyl-epothilon A (werden in 1 ml Dichlorethan gelöst, mit 2 ml (0.013 mol)

1,8-Diazabicyclo[5.4.0]undec-7-en (DBU) versetzt und 12 Stunden bei 90 °C gerührt.

Zur Aufarbeitung wird das Reaktionsgemisch mit 1 M Natriumdihydrogenphosphat-Puffer pH 4.5 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des aus zwei Verbindungen bestehenden Rohproduktes erfolgt mittels präparativer Schichtchromatographie (Laufmittel: Dichlormethan/Aceton, 90 : 10).
Ausbeute: 7 mg (15 %)

### Substanzcode

R_{f} (Dichlormethan/Aceton, 90 : 10): 0.62
IR (Film): ny = 2951 (m, br, Sch), 1723 (vs), 1644 (w, br, Sch), 1468 (w), 1377 (w), 1271 (m, br, Sch), 1179 (s), 987 (m, br, Sch), 735 (w, br, Sch) cm⁻¹.
UV (Methanol): lambdaₘₐₓ (lg epsilon) = 210 (4.44) nm.
MS (20/70 eV): m/e (%) = 503 (68 [M⁺]), 408 (58), 390 (32), 334 (25), 316 (34), 220 (21), 206 (27), 194 (20), 181 (33), 164 (100), 151 (34), 139 (28), 113 (20), 96 (82), 81 (33), 67 (24), 55 (26), 43 (22).
Hochauflösung: C₂₇H₃₇O₆NS ber.: 503.2342 für [M⁺]
gef.: 503.2303

### Beispiel 10:

### Verbindung 6c

5 mg (0.009 mmol) 3,7-Di-O-acetyl-epothilon werden in 1 ml Methanol gelöst, mit 150 µl einer ammoniakalischen Methanollösung (2 mmol NH₃/ml Methanol) versetzt und über Nacht bei 50 °C gerührt.

Zur Aufarbeitung wird das Lösungsmittel im Vakuum entfernt. Die Reinigung des Rohproduktes erfolgt mit Hilfe der präparativen Schichtchromatographie (Laufmittel: Toluol/Methanol, 90 : 10).
Ausbeute: 3 mg (67 %)
R_{f} (Dichlormethan/Aceton, 90 : 10): 0.55
IR (Film): ny = 2934 (s, b, Sch), 1719 (vs, b, Sch), 1641 (m), 1460 (m, Sch), 1372 (s, Sch), 1237 (vs, b, Sch), 1179 (s, Sch), 1020 (s), 963 (s, Sch), 737 (vs) cm⁻¹.
UV (Methanol): lambdaₘₐₓ (lg epsilon) = 210 (4.33) nm.
MS (20/70 eV): m/e (%) = 517 (57 [M⁺]), 422 (58), 318 (31), 194 (20), 181 (34), 166 (100), 151 (31), 96 (96), 81 (32), 69 (27), 55 (29), 43 (69).
Hochauflösung: C₂₈H₃₉O₆NS ber.: 517.2498 für [M⁺]
gef.: 517 2492

### Beispiel 11:

### Verbindung 7a

20 mg (0.041 mmol) Epothilon werden in 0.5 ml Methanol gelöst, mit 0.5 ml 1 N Natronlauge versetzt und 5 Minuten bei Raumtemperatur gerührt.

Zur Aufarbeitung wird das Reaktionsgemisch mit 1 M Phosphatpuffer pH 7 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes erfolgt mit Hilfe der präparativen Schichtchromatographie (Laufmittel: Dichlormethan/Methanol, 85 : 15).
Ausbeute: 11 mg (52 %)
R_{f} (Dichlormethan/Methanol, 85 : 15): 0.92
IR (Film): ny = 3438 (s, br, Sch), 2971 (vs, br, Sch), 1703 (vs), 1507 (m), 1460 (s, Sch), 1383 (m, Sch), 1254 (w), 1190 (w, br, Sch), 1011 (w, br, Sch), 866 (w, br), 729 (s) cm⁻¹
MS (20/70 eV): m/e (%) = 423 (0.1 [M⁺]), 323 (4), 168 (89), 140 (100), 85 (31), 57 (67).
Hochauflösung: C₂₃H₃₇O₄NS ber.: 423.2443 für [M⁺]
gef.: 423.2410

### Beispiel 12:

### Verbindung 7b

5 mg (0.009 mmol) 7-O-Acetyl-epothilon werden in 1 ml Methanol gelöst, mit 200 µl einer ammoniakalischen Methanollösung (2 mmol NH₃/ml Methanol) versetzt und zwei Tage bei 50 °C gerührt. Zur Aufarbeitung wird das Lösungsmittel im Vakuum entfernt. Die Reinigung des Rohproduktes erfolgt mit Hilfe der präparativen Schichtchromatographie (Laufmittel: Toluol/Methanol, 90 : 10).
Ausbeute: 3 mg (59 %)
R_{f} (Dichlormethan/Methanol, 90 : 10): 0.63
IR (Film): ny = 3441 (m, b, Sch), 2946 (s, Sch), 1732 (vs), 1600 (w), 1451 (m), 1375 (m), 1246 (s, b, Sch), 1013 (m, b, Sch) cm⁻¹
UV (Methanol): lambdaₘₐₓ (lg epsilon) = 211 (3.75), 247 (3.59) nm.
MS (20/70 eV) : m/e (%) = 567 (1 [M⁺]), 465 (4), 422 (7), 388 (5), 194 (5), 182 (7), 168 (65), 164 (17), 140 (100), 97 (10), 71 (22), 43 (27).
Hochauflösung: C₂₉H₄₅O₈NS ber.: 567.2866 für [M⁺]
gef.: 567.2849

### Beispiel 13:

50 mg Epothilon A werden in 20 µl Dimethylsulfoxid angelöst und mit 30 ml Phosphatpuffer (pH 7,1, 30 mM) verdünnt. Nach Zugabe von 5 mg Schweineleberesterase (Fa. Boehringer Mannheim) wird 2 Tage bei 30 °C gerührt. Man säuert mit 2 N HCl auf pH 5 an und extrahiert die Epothilonsäure 7 mit Ethylacetat. Die organische Phase wird mit Natriumsulfat getrocknet, im Vakuum zur Trockne eingedampft. Ausbeute 48 mg (96 %).

### Beispiel 14:

48 mg Epothilonsäure 7 werden in 6 ml THF abs. gelöst und unter Rühren mit 40 µl Triethylamin und 16 µl 2,4,6-Trichlorbenzoylchlorid versetzt. Nach 15 min wird vom Niederschlag abfiltriert und innerhalb von 15 min unter schnellem Rühren in eine siedende Lösung von 20 mg 4-Dimethylaminopyridin in 200 ml Toluol abs. getropft. Nach weiteren 10 min wird im Vakuum eingedampft und der Rückstand zwischen Ethylacetat/Citratpuffer (pH 4) verteilt. Der Eindampfrückstand der organischen Phase ergibt nach präparativer HPLC Trennung 15 mg Epothilon A.

### Beispiel 15:

### Epothilone C und D als Ausgangsverbindungen

### A. Produktionsstamm und Kulturbedingungen entsprechend dem Epothilon Basispatent.

### B. Produktion mit DSM 6773

75 l Kultur werden wie im Basispatent beschrieben angezogen und zum Animpfen eines Produktionsfermenters mit 700 l Produktionsmedium aus 0.8 % Stärke, 0.2 % Glukose, 0.2 % Soyamehl, 0.2 % Hefeextrakt, 0.1 % CaCl₂ x 2H₂O, 0.1 % MgSO₄ x 7H₂O, 8 mg/l Fe-EDTA, pH = 7.4 und optional 15 l Adsorberharz Amberlite XAD-16 verwendet. Die Fermentation dauert 7 - 10 Tage bei 30 C, Belüftung mit 2 m³ Luft/h. Durch Regulierung der Drehzahl wird der pO₂ bei 30 % gehalten.

### C. Isolierung

Das Adsorberharz wird mit einem 0.7 m², 100 mesh Prozeßfilter von der Kultur abgetrennt und durch Waschen mit 3 Bettvolumen Wasser/Methanol 2:1 von polaren Begleitstoffen befreit. Durch Elution mit 4 Bettvolumen Methanol wird ein Rohextrakt gewonnen, der i. Vak. bis zum Auftreten der Wasserphase eingedampft wird. Diese wird dreimal mit dem gleichen Volumen Ethylacetat extrahiert. Eindampfen der organischen Phase ergibt 240 g Rohextrakt, der zwischen Methanol und Heptan verteilt wird, um lipophile Begleitstoffe abzutrennen. Aus der Methanolphase werden durch Eindampfen i. Vak. 180 g Raffinat gewonnen, das in drei Portionen über Sephadex LH-20 (Säule 20 x 100 cm, 20 ml/min Methanol) fraktioniert wird. Die Epothilone sind in der mit 240 - 300 min Retentionszeit eluierten Fraktion von insgesamt 72 g enthalten. Zur Trennung der Epothilone wird in drei Portionen an Lichrosorb RP-18 (15 µm, Säule 10 x 40 cm, Laufmittel 180 ml/min Methanol/Wasser 65:35) chromatographiert. Nach Epothilon A und B werden mit Rₜ = 90-95 min Epothilon C und 100-110 min Epothilon D eluiert und nach Eindampfen i. Vak. in einer Ausbeute von jeweils 0.3 g als farblose Öle gewonnen.

### D. Physikalische Eigenschaften

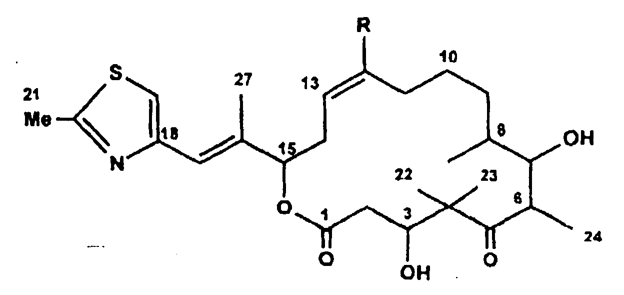

Epothilon C R = H
Epothilon D R = CH₃

### Epothilon C

C₂₆H₃₉NO₅S [477]
ESI-MS: (positiv Ionen): 478.5 für [M+H]⁺
1H und 13C siehe NMR-Tabelle
DC:R_{f} = 0,82
DC-Alufolie 60 F 254 Merck, Laufmittel: Dichlormethan/Methanol = 9:1
Detektion: UV-Löschung bei 254 nm. Ansprühen mit Vanillin-Schwefelsäure-Reagenz, blau-graue Anfärbung beim Erhitzen auf 120 °C.
HPLC:Rₜ = 11,5 min
Säule: Nucleosil 100 C-18 7µm, 125 x 4 mm
Laufmittel: Methanol/Wasser = 65:35
Fluß: 1ml/min
Detection: Diodenarray

### Epothilon D

C₂₇H₄₁NO₅S [491]
ESI-MS: (positiv Ionen): 492,5 für [M+H]⁺
1H und 13C siehe NMR-Tabelle
DC:R_{f} = 0,82
DC-Alufolie 60 F 254 Merck, Laufmittel: Dichlormethan/Methanol = 9:1
Detektion: UV-Löschung bei 254 nm. Ansprühen mit Vanillin-Schwefelsäure-Reagenz, blau-graue Anfärbung beim Erhitzen auf 120 °C.
HPLC:Rₜ = 15,3 min
Säule: Nucleosil 100 C-18 7µm, 125 x 4 mm
Laufmittel: Methanol/Wasser = 65:35
Fluß: 1ml/min
Detection: Diodenarray

**Tabelle:**

| ¹H-und ¹³C-NMR Daten von Epothilon C und Epothilon D in [D₆]DMSO bei 300 MHz | | | | | | |
|---|---|---|---|---|---|---|
| Epothilon C | | | | Epothilon D | | |
| H-Atom | δ (ppm) | C-Atom | δ (ppm) | δ (ppm) | C-Atom | δ (ppm) |
| | | 1 | 170.3 | | 1 | 170.1 |
| 2-Ha | 2.38 | 2 | 38.4 | 2.35 | 2 | 39.0 |
| 2-Hb | 2.50 | 3 | 71.2 | 2.38 | 3 | 70.8 |
| 3-H | 3.97 | 4 | 53.1 | 4.10 | 4 | 53.2 |
| 3-OH | 5.12 | 5 | 217.1 | 5.08 | 5 | 217.4 |
| 6-H | 3.07 | 6 | 45.4 | 3.11 | 6 | 44.4 |
| 7-H | 3.49 | 7 | 75.9 | 3.48 | 7 | 75.5 |
| 7-OH | 4.46 | 8 | 35.4 | 4.46 | 8 | 36.3 |
| 8-H | 1.34 | 9 | 27.6 | 1.29 | 9 | 29.9 |
| 9-Ha | 1.15 | 10 | 30.0 | 1.14 | 10 | 25.9 |
| 9-Hb | 1.40 | 11 | 27.6 | 1.38 | 11 | 31.8* |
| 10-Ha | 1.15* | 12 | 124.6 | 1.14* | 12 | 138.3 |
| 10-Hb | 1.35* | 13 | 133.1 | 1.35* | 13 | 120.3 |
| 11-Ha | 1.90 | 14 | 31.1 | 1.75 | 14 | 31.6* |
| 11-Hb | 2.18 | 15 | 76.3 | 2.10 | 15 | 76.6 |
| 12-H | 5.38** | 16 | 137.3 | | 16 | 137.2 |
| 13-H | 5.44** | 17 | 119.1 | 5.08 | 17 | 119.2 |
| 14-Ha | 2.35 | 18 | 152.1 | 2.30 | 18 | 152.1 |
| 14-Hb | 2.70 | 19 | 117.7 | 2.65 | 19 | 117.7 |
| 15-H | 5.27 | 20 | 164.2 | 5.29 | 20 | 164.3 |
| 17-H | 6.50 | 21 | 18.8 | 6.51 | 21 | 18.9 |
| 19-H | 7.35 | 22 | 20.8 | 7.35 | 22 | 19.7 |
| 21-H₃ | 2.65 | 23 | 22.6 | 2.65 | 23 | 22.5 |
| 22-H₃ | 0.94 | 24 | 16.7 | 0.90 | 24 | 16.4 |
| 23-H₃ | 1.21 | 25 | 18.4 | 1.19 | 25 | 18.4 |
| 24-H₃ | 1.06 | 27 | 14.2 | 1.07 | 26 | 22.9 |
| 25-H₃ | 0.90 | | | 0.91 | 27 | 14.1 |
| 26-H₃ | | | | 1.63 | | |
| 27-H₃ | 2.10 | | | 2.11 | | |

| | | | | | | |
|---|---|---|---|---|---|---|
| *, ** Zuordnung vertauschbar | | | | | | |

### Beispiel 16:

### Epothilon A und 12,13-Bisepi-epothilon A aus Epothilon C

50 mg Epothilon A werden in 1.5 ml Aceton gelöst und mit 1.5 ml einer 0.07 molaren Lösung von Dimethyldioxiran in Aceton versetzt. Nach 6 Stunden Stehen bei Raumtemperatur wird i. Vak. eingedampft und durch präparative HPLC an Kieselgel (Laufmittel: Methyl-tert.butylether/Petrolether/Methanol 33:66:1) getrennt.

### Ausbeute:

25 mg Epothilon A, Rₜ = 3,5 min (analyt. HPLC, 7 µm, Säule 4 x 250 mm, Laufmittel s. o., Fluß 1.5 ml/min)
und
20 mg 12,13-Bisepi-epothilon A, Rₜ = 3.7 min, ESI-MS (pos. Ionen)
m/z = 494 [M+H]⁺
¹H-NMR in [D₄] Methanol, ausgewählte Signale: delta = 4.32 (3-H), 3.79 (7-H), 3.06 (12-H), 3.16 (13-H), 5.54 (15-H), 6.69 (17-H), 1.20 (22-H), 1.45 (23-H). 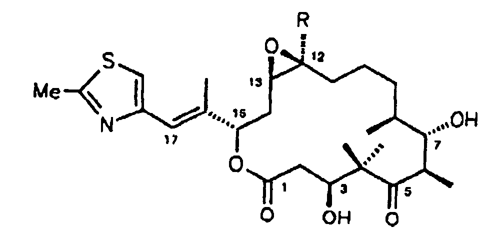 12,13-Bisepi-epothilon A R = H
1. Epothilonderivat der Formel 1 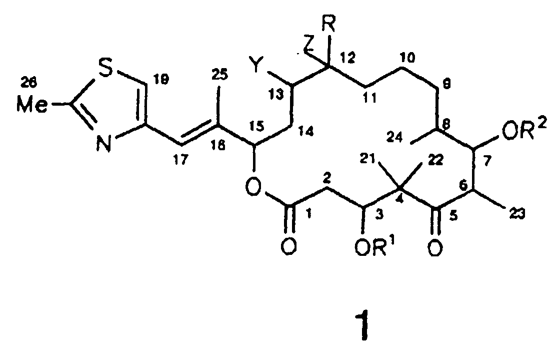 wobei R = H, C₁₋₄-Alkyl; R¹, R² = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl; und es sich bei den in den Resten enthaltenen Alkyl- bzw. Acylgruppen um gradkettige oder verzweigte Reste handelt, und Y und Z entweder gleich oder verschieden sind und jeweils für Wasserstoff, Halogen, Pseudohalogen, OH, O-(C₁₋₆)-Acyl, O-(C₁₋ ₆)-Alkyl oder O-Benzoyl stehen oder gemeinsam das O-Atom eines Epoxids oder eine der C-C-Bindungen einer C=C-Doppelbindung bilden, wobei Epothilon A und B ausgenomen sind.
2. Epothilonderivat der Formel 2 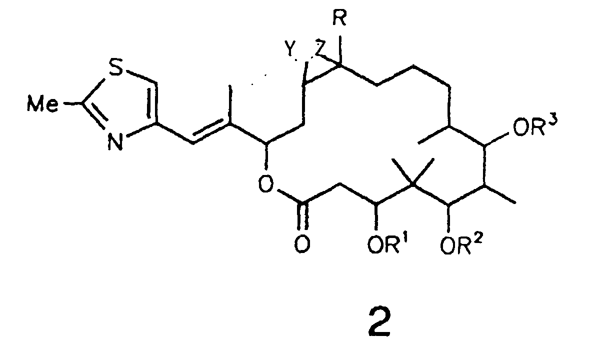 wobei R = H, C₁₋₄-Alkyl; R¹, R², R³ = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl; es sich bei den in den Resten enthaltenen Alkyl- bzw. Acylgruppen um gradkettige oder verzweigte Reste handelt; und Y und Z die Bedeutungen gemäß Anspruch 1 besitzen.
3. Epothilonderivat der Formel 3 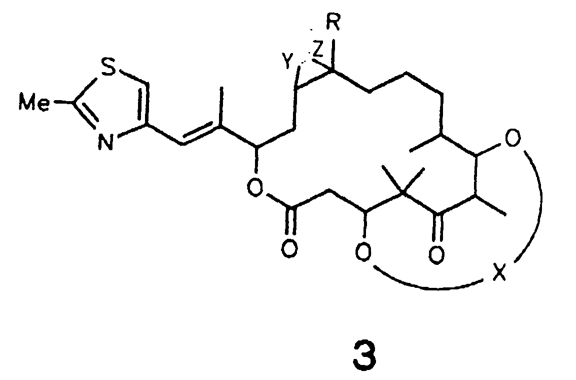 wobei R = H, C₁₋₄-Alkyl; R¹, R² = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl; es sich bei den in den Resten enthaltenen Alkyl- bzw. Acylgruppen um gradkettige oder verzweigte Reste handelt, und X allgemein für -C(O)-, -C(S)-, -S(O)-, -CR¹R²- und -SiR₂- steht, wobei R, R¹ und R² die Bedeutung haben wie oben angegeben und R¹ und R² auch zusammen eine Alkylengruppe mit 2 bis 6 Kohlenstoffatomen bilden können; und Y und Z die Bedeutungen gemäß Anspruch 1 besitzen.
4. Epothilonderivat der Formel 4 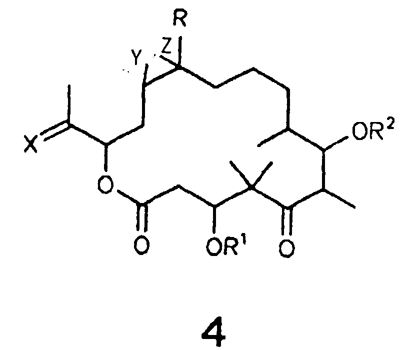 wobei R = H, C₁₋₄-Alkyl; R¹, R², R³, R⁴, R⁵ = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl; es sich bei den in den Resten enthaltenen Alkyl- bzw. Acylgruppen um gradkettige oder verzweigte Reste handelt, X Sauerstoff, NOR³, N-NR⁴R⁵, und N-NHCONR⁴R⁵ bedeutet, wobei die Reste R³ bis R⁵ die oben angegebene Bedeutung haben und R⁴ und R⁵ auch zusammen eine Alkylengruppe mit 2 bis 6 Kohlenstoffatomen bilden können; und
   Y und Z die Bedeutungen gemäß Anspruch 1 besitzen.
5. Epothilonderivat der Formel 5 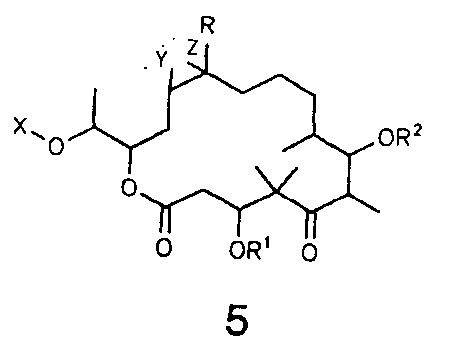 wobei R = H, C₁₋₄-Alkyl; R¹, R² = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl; es sich bei den in den Resten enthaltenen Alkyl- bzw. Acylgruppen um gradkettige oder verzweigte Reste handelt, und X Wasserstoff, C₁₋₁₈-Alkyl, C₁₋₁₈-Acyl, Benzyl, Benzoyl und Cinnamoyl bedeutet und
   Y und Z die Bedeutungen gemäß Anspruch 1 besitzen.
6. Epothilonderivat der Formel 6 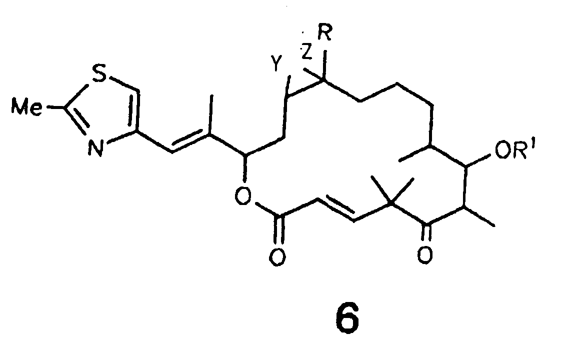 wobei R = H, C₁₋₄-Alkyl und R¹ = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl ist; es sich bei den in den Resten enthaltenen Alkyl- bzw. Acylgruppen um gradkettige oder verzweigte Reste handelt; und
   Y und Z die Bedeutungen gemäß Anspruch 1 besitzen.
7. Epothilonderivat der Formel 7 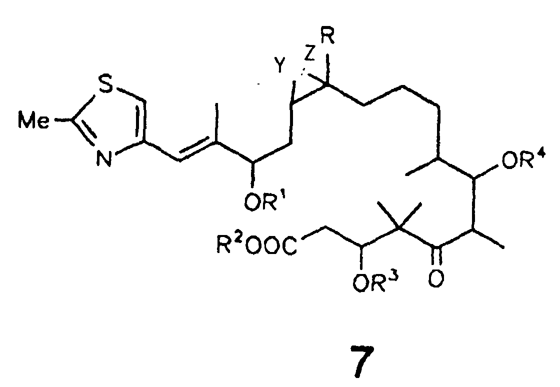 wobei R = H, C₁₋₄-Alkyl und R¹, R², R³, R⁴ = H, C₁₋₆-Alkyl, C₁₋₆-Acyl, Benzoyl, C₁₋₄-Trialkylsilyl, Benzyl, Phenyl, C₁₋₆-Alkoxy, C₆-Alkyl-, Hydroxy- und halogensubstituiertes Benzyl bzw. Phenyl; es sich bei den in den Resten enthaltenen Alkyl- bzw. Acylgruppen um gradkettige oder verzweigte Reste handelt; und Y und Z die Bedeutungen gemäß Anspruch 1 besitzen.
8. Verfahren zur Herstellung eines Epothilonderivats der Formel 7 gemäß Anspruch 7, **dadurch *gekennzeichnet*, daß** man Epothilon A, Epothilon B, ein 3-OH-geschütztes Derivat derselben oder ein 7-OH-geschütztes Derivat derselben
   (a) enzymatisch hydrolysiert, insbesondere mit einer Esterase oder Lipase, oder
   (b) in alkalischem Medium hydrolysiert, insbesondere mit Natriumhydroxid in einem Methanol/Wasser-Gemisch,
   und das Epothilonderivat der Formel 7 gewinnt und isoliert.
9. Verfahren zur Herstellung eines Epothilonderivats der Formel 2 gemäß Anspruch 2, **dadurch *gekennzeichnet,* daß** man ein Epothilonderivat der Formel 7 gemäß Anspruch 7 oder als Produkt des Verfahrens gemäß Anspruch 8
   (a) nach der Yamaguchi-Methode oder
   (b) nach der Corey-Methode oder
   (c) nach der Kellogg-Methode
   in das Epothilonderivat der Formel 2 umwandelt und dieses Umwandlungsprodukt isoliert.
10. Verfahren zur Herstellung von Epothilon A und/oder 12,13-Bisepi-epothilon A, **dadurch gekennzeichnet, daß** man Epothilon C epoxidiert, insbesondere mit Dimethyldioxiran oder einer Persäure.
11. Verfahren zur Herstellung von Epothilon B und/oder 12,13-Bisepi-epothilon B, **dadurch gekennzeichnet, daß** man Epothilon D epoxidiert, insbesondere mit Dimethyldioxiran oder einer Persäure.
12. Mittel für den Pflanzenschutz in der Landwirtschaft und Forstwirtschaft und/oder im Gartenbau, bestehend aus einem oder mehreren der Verbindungen gemäß einem der vorangehenden Ansprüche oder einer oder mehreren dieser Verbindungen neben einem oder mehreren üblichen Träger(n) und/oder Verdünnungsmittel(n).
13. Therapeutisches Mittel, insbesondere zum Einsatz als Cytostatikum, bestehend aus einer oder mehrerer der Verbindungen nach einem oder mehreren der Ansprüche 1 bis 7 oder einer oder mehrerer der Verbindungen nach einem oder mehreren der Ansprüche 1 bis 7 neben einem oder mehreren üblichen Träger(n) und/oder Verdünnungsmittel(n).

## Patentansprüche

1. Epothilonderivat der Formel 1 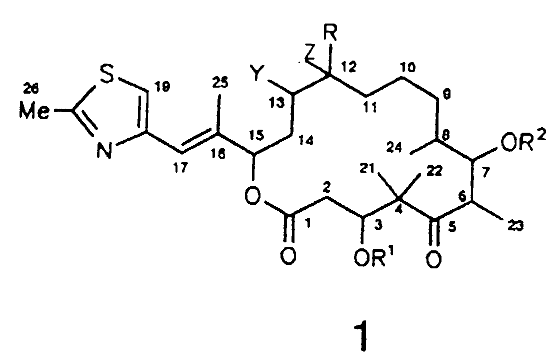 wobei R = H, C₁₋₄-Alkyl; R¹, R² = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl; und es sich bei den in den Resten enthaltenen Alkyl- bzw. Acylgruppen um gradkettige oder verzweigte Reste handelt, und Y und Z entweder gleich oder verschieden sind und jeweils für Wasserstoff, Halogen, Pseudohalogen, OH, O-(C₁₋₆)-Acyl, O-(C₁₋ ₆)-Alkyl oder O-Benzoyl stehen oder gemeinsam das O-Atom eines Epoxids , wobei Epothilon A und B ausgenomen sind.

2. Epothilonderivat der Formel 2 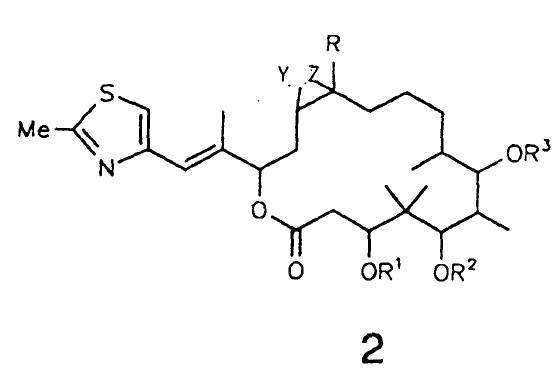 wobei R = H, C₁₋₄-Alkyl; R¹, R², R³ = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl; es sich bei den in den Resten enthaltenen Alkyl- bzw. Acylgruppen um gradkettige oder verzweigte Reste handelt; und Y und Z die Bedeutungen gemäß Anspruch 1 besitzen.

3. Epothilonderivat der Formel 4 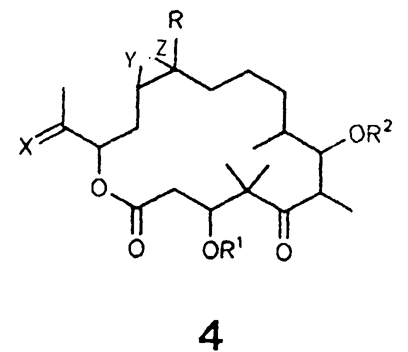 wobei R = H, C₁₋₄-Alkyl; R¹, R², R³, R⁴, R⁵ = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl; es sich bei den in den Resten enthaltenen Alkyl- bzw. Acylgruppen um gradkettige oder verzweigte Reste handelt, X Sauerstoff, NOR³, N-NR⁴R⁵, und N-NHCONR⁴R⁵ bedeutet, wobei die Reste R³ bis R⁵ die oben angegebene Bedeutung haben und R⁴ und R⁵ auch zusammen eine Alkylengruppe mit 2 bis 6 Kohlenstoffatomen bilden können; und
Y und Z die Bedeutungen gemäß Anspruch 1 besitzen.

4. Epothilonderivat der Formel 5 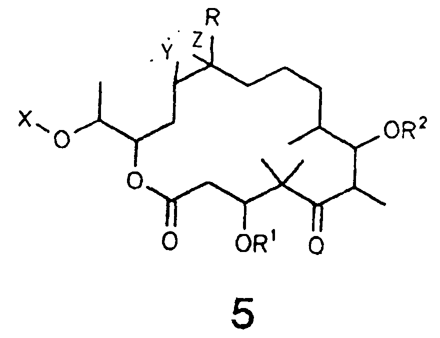 wobei R = H, C₁₋₄-Alkyl; R¹, R² = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl; es sich bei den in den Resten enthaltenen Alkyl- bzw. Acylgruppen um gradkettige oder verzweigte Reste handelt, und X Wasserstoff, C₁₋₁₈-Alkyl, C₁₋₁₈-Acyl, Benzyl, Benzoyl und Cinnamoyl bedeutet und
Y und Z die Bedeutungen gemäß Anspruch 1 besitzen.

5. Epothilonderivat der Formel 7 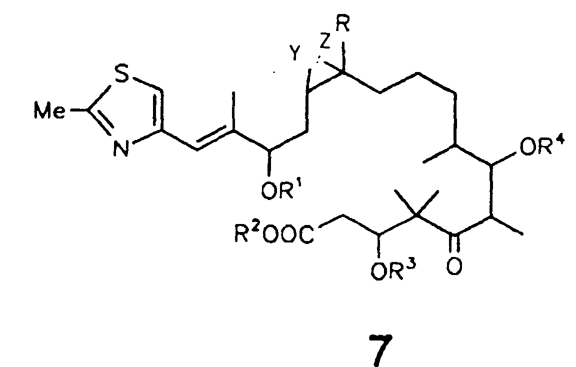 wobei R = H, C₁₋₄-Alkyl und R¹, R², R³, R⁴ = H, C₁₋₆-Alkyl, C₁₋₆-Acyl, Benzoyl, C₁₋₄-Trialkylsilyl, Benzyl, Phenyl, C₁₋₆-Alkoxy, C₆-Alkyl-, Hydroxy- und halogensubstituiertes Benzyl bzw. Phenyl; es sich bei den in den Resten enthaltenen Alkyl- bzw. Acylgruppen um gradkettige oder verzweigte Reste handelt; und
Y und Z entweder gleich oder verschieden sind und jeweils für Wasserstoff, Halogen, Pseudohalogen, OH, O-(C₁₋₆)-Acyl, O-(C₁₋₆)-Alkyl oder O-Benzoyl stehen oder gemeinsam das O-Atom eines Epoxids bilden.

6. Verfahren zur Herstellung eines Epothilonderivats der Formel 7 gemäß Anspruch 5, **dadurch *gekennzeichnet,* daß** man Epothilon A, Epothilon B, ein 3-OH-geschütztes Derivat derselben oder ein 7-OH-geschütztes Derivat derselben
(a) enzymatisch hydrolysiert, insbesondere mit einer Esterase oder Lipase, oder
(b) in alkalischem Medium hydrolysiert, insbesondere mit Natriumhydroxid in einem Methanol/Wasser-Gemisch,
und das Epothilonderivat der Formel 7 gewinnt und isoliert.

7. Verfahren zur Herstellung eines Epothilonderivats der Formel 1 gemäß Anspruch 1, **dadurch *gekennzeichnet,* daß** man ein Epothilonderivat der Formel 7 gemäß Anspruch 5 oder als Produkt des Verfahrens gemäß Anspruch 6
(a) nach der Yamaguchi-Methode oder
(b) nach der Corey-Methode oder
(c) nach der Kellogg-Methode
in das Epothilonderivat der Formel 1 umwandelt und dieses Umwandlungsprodukt isoliert.

8. Mittel für den Pflanzenschutz in der Landwirtschaft und Forstwirtschaft und/oder im Gartenbau, bestehend aus einem oder mehreren der Verbindungen gemäß einem der vorangehenden Ansprüche oder einer oder mehreren dieser Verbindungen neben einem oder mehreren üblichen Träger(n) und/oder Verdünnungsmittel(n).

9. Therapeutisches Mittel, insbesondere zum Einsatz als Cytostatikum, bestehend aus einer oder mehrerer der Verbindungen nach einem oder mehreren der Ansprüche 1 bis 5 oder einer oder mehrerer der Verbindungen nach einem oder mehreren der Ansprüche 1 bis 5 neben einem oder mehreren üblichen Träger(n) und/oder Verdünnungsmittel(n).
